# EUROPEAN PATENT SPECIFICATION

(11) **EP 3 065 711 B1**
(45) Date of publication and mention of the grant of the patent: **04.09.2024**
(21) Application number: 14860556.1
(22) Date of filing: 23.10.2014
(51) Int. Cl.: A61K 9/14, A61K 47/00, A61K 48/00, A61K 9/51, A61K 9/00, C12N 15/87

(54) **DRY TRANSFECTION COMPOSITIONS AND METHODS FOR MAKING AND USING THE SAME**
TROCKENE TRANSFEKTIONSZUSAMMENSETZUNGEN UND VERFAHREN ZUR HERSTELLUNG UND VERWENDUNG DAVON
COMPOSITIONS DE TRANSFECTION SÈCHES ET PROCÉDÉS DE FABRICATION ET D'UTILISATION DE CELLES-CI

(30) Priority: 05.11.2013 US 201361900232 P
(43) Date of publication of application: 14.09.2016
(73) Proprietor: Takara Bio USA, Inc., Mountain View, CA 94043 (US)
(72) Inventor: QUINN, Thomas Patrick, Sunnyvale, California 94086 (US); MITRA, Sayantan, Mountain View, California 94043 (US)
(74) Representative: Carpmaels & Ransford LLP
(86) International application number: PCT/US2014/062011
(87) International publication number: WO 2015/069469

(56) References cited:
- WO-A2-2005/055979
- WO-A2-2005/055979
- US-A1- 2002 131 951
- US-A1- 2006 083 711
- US-A1- 2011 008 894
- US-B2- 6 998 115
- US-B2- 7 427 394
- SUNSHINE J C ET AL: "Poly(beta-Amino Ester)-Nanoparticle Mediated Transfection of Retinal Pigment Epithelial Cells In Vitro and In Vivo", PLOS ONE, vol. 7, no. 5, 21 May 2012 (2012-05-21), pages E37543-1 - 11, XP002746169, ISSN: 1932-6203, [retrieved on 20120521], DOI: 10.1371/JOURNAL.PONE.0037543

## Description

### CROSS-REFERENCE TO RELATED APPLICATIONS

Pursuant to 35 U.S.C. § 119 (e), this application claims priority to the filing date of the United States Provisional Patent Application Serial No. 61/900,232, filed November 5, 2013.

### INTRODUCTION

Being able to effectively introduce foreign molecules, such as DNA, siRNA, protein, etc., into biological cells is of great importance in biology and biotechnology. Transfection refers to the transfer of nucleic acids into a cell. Effective transfection of biological cells with nucleic acid molecules is an essential prerequisite in elucidating how genes function in complex cellular context and how their activities could be modulated for therapeutic intervention. Gell transfection is routinely used in fundamental biology research and pharmaceutical development.

Nucleic acids can be delivered to cells both in vitro and in vivo by pre-complexing the nucleic acids with charged lipids, lipidoids, peptides, polymers or mixtures thereof. Such transfection reagents are commercially available, and are widely used for delivering nucleic acids to cells in culture. Cells exposed to transfection reagent-nucleic acid complexes may internalize these complexes by endocytosis or other means.

### SUMMARY

Dry, e.g., lyophilized, polymeric transfection agent compositions are provided. The dry compositions include a polymeric transfection agent and a buffer. In some instances, the compositions further include one or more nucleic acids. Also provided are methods of making and using the compositions, as well as kits including the compositions.

### BRIEF DESCRIPTION OF THE FIGURES

FIG. 1 depicts results from fluorescent activated cell sorting (FACS) analysis of transfected cells at 72hrs post transfection to determine transfection efficiency. % positive cells are represented as a bar graph (left Y-axis) and mean fluorescent intensity (MFI) is represented as a line graph (right Y-axis).
FIG. 2 depicts results from quantitative RT-PCR (qRT-PCR) of lentiviral stocks produced with lyophilized packaging components to determine the amounts of produced lentivirus.
FIG. 3 depicts results from determining the infectious units per ml (IFU/ml) of lentiviral stocks produced with lyophilized packaging components to determine the titer of produced lentivirus.
FIG. 4 depicts results from determining the transfection efficiency (Top panel: % positive cells and MFI) and copies/ml of virus (Bottom panel: qRT-PCR) of lentiviral stocks produced with lyophilized packaging components when tested again with additional samples (N=8 liquid; N=13 dry).
FIG. 5 provides of view of a multi-well substrate according to one embodiment of the invention.

### DETAILED DESCRIPTION

Dry, e.g., lyophilized, polymeric transfection agent compositions are provided. The dry compositions include a polymeric transfection agent and a buffer. In some instances, the compositions further include one or more nucleic acids. Also provided are methods of making and using the compositions, as well as kits including the compositions.

Before the methods of the present disclosure are described in greater detail, it is to be understood that the methods are not limited to particular embodiments described, as such may, of course, vary. It is also to be understood that the terminology used herein is for the purpose of describing particular embodiments only, and is not intended to be limiting, since the scope of the methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the methods. The upper and lower limits of these smaller ranges may independently be included in the smaller ranges and are also encompassed within the methods, subject to any specifically excluded limit in the stated range. Where the stated range includes one or both of the limits, ranges excluding either or both of those included limits are also included in the methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which the methods belong. Although any methods similar or equivalent to those described herein can also be used in the practice or testing of the methods, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the methods, which are, for clarity, described in the context of separate embodiments, may also be provided in combination in a single embodiment. Conversely, various features of the methods, which are, for brevity, described in the context of a single embodiment, may also be provided separately or in any suitable sub-combination. All combinations of the embodiments are specifically embraced by the present invention and are disclosed herein just as if each and every combination was individually and explicitly disclosed, to the extent that such combinations embrace operable processes and/or devices/systems/kits. In addition, all sub-combinations listed in the embodiments describing such variables are also specifically embraced by the present methods and are disclosed herein just as if each and every such sub-combination was individually and explicitly disclosed herein.

As will be apparent to those of skill in the art upon reading this disclosure, each of the individual embodiments described and illustrated herein has discrete components and features which may be readily separated from or combined with the features of any of the other several embodiments without departing from the scope of the present methods. Any recited method can be carried out in the order of events recited or in any other order which is logically possible.

### DRIED TRANSFECTION AGENT COMPOSITIONS

As summarized above, aspects of the invention include dried polymeric transfection agent compositions. As the compositions are dried, they are substantially, if not completely, free of uncombined water. By substantially free of uncombined water is meant that the compositions include 0.1 wt.% or less, such as 0.01 wt.% or less, including 0.001 wt.% or less of uncombined water, including, in some instances, no uncombined water. The dried compositions may be particulate compositions. A particulate composition is a composition made up of particles, where the particles may vary in size, ranging in some instances from 5 to 1000 nm, such as 10 to 500 nm and including 50 to 400 nm.

In some instances, the dried polymeric transfection agents are lyophilized compositions. By lyophilized is meant that the composition has been subjected to freeze drying conditions, such that the composition may be described as a freeze-dried composition. Examples of suitable freeze drying conditions are provided below.

The dried compositions described herein include a polymeric transfection agent. By polymeric transfection agent is meant one or more polymers that have transfection activity, i.e., when combined with a nucleic acid and contacted with a cell, they enhance transportation of the nucleic acid into the cell. The magnitude of enhancement may vary, where in some instances the magnitude of enhancement is 2 fold or greater, such as 3 or 5 fold or greater, including 10-fold or greater, e.g., as compared to a suitable control in which a transfection agent is not present.

As the transfection agents are polymeric, they are large molecules made up of multiple repeats of smaller units, i.e., monomeric residues, In some instances, the polymeric transfection agents are heterpolymers, and in some instances are poly(βamino esters) and salts and derivatives thereof. Polymeric transfection agents of interest are biodegradable and biocompatible. In some instances, the polymers of the polymeric transfection agents have one or more tertiary amines in the backbone of the polymer, such as one or two tertiary amines per repeating backbone unit. The polymers may also be co-polymers in which one of the components is a poly(β-amino ester). A polymer of the invention is represented by either of the formula below: 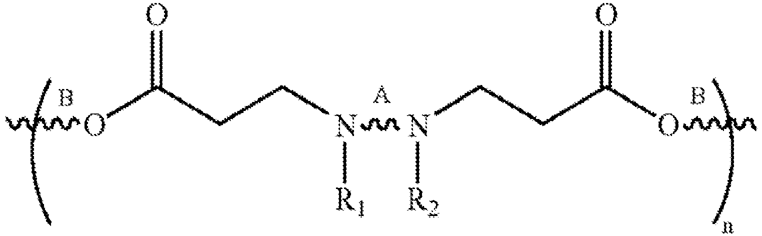 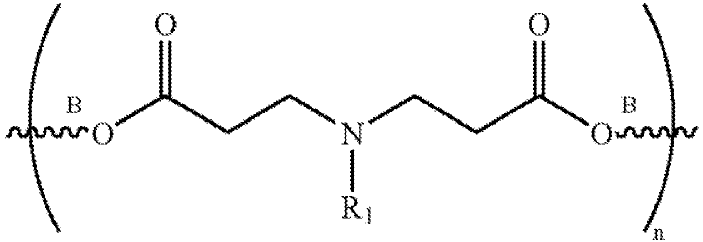 where A and B are linkers which may be any substituted or unsubstituted, branched or unbranched chain of carbon atoms or heteroatoms. The molecular weights of the polymers may range from 1000 g/mol to 20,000 g/mol, such as from 5000 g/mol to 15,000 g/mol. In certain embodiments, B is an alkyl chain of one to twelve carbons atoms. In other embodiments, B is a heteroaliphatic chain containing a total of one to twelve carbon atoms and heteroatoms. The groups R₁ and R₂ may be any of a wide variety of substituents. In certain embodiments, R₁ and R₂ may contain primary amines, secondary amines, tertiary amines, hydroxyl groups, and alkoxy groups. In certain embodiments, the polymers are amine-terminated; and in other embodiments, the polymers are acrylated terminated. In some instances, the groups R₁ and/or R₂ form cyclic structures with the linker A. Polymers containing such cyclic moieties have the characteristic of being more soluble at lower pH. Polymers of interest are those that are insoluble in aqueous solutions at physiologic pH (pH 7.2-7.4) and are soluble in aqueous solutions below physiologic pH (pH<7.2). Other polymers of interest are those that are soluble in aqueous solution at physiologic pH (pH 7.2-7.4) and below. In some instances, the polymeric transfection agent is a polymer described in U.S Patent Nos. 6,998,115 and 7,427,394. One specific transfection agent of interest described in these patents is the XFECT^{™} transfection agent (Clontech, Mountain View CA).

The amount of polymeric transfection agent in the dried compositions may vary. While the amount of polymeric transfection agent in the composition may vary (and may be based on a number of factors such as, but not limited to, particular type of transfection agent, type of nucleic acid, type of target cell, other components in the composition, etc.), in some instances the amount of polymeric transfection agent present in the dried compositions ranges from 5 to 75, such as 5 to 50 and including 10 to 30, e.g., 25 to 27.5 wt. %.

In addition to the polymeric transfection agent, the dried compositions described herein also include a buffer component. The buffer component may vary, where buffers of interest include, but are not limited to: potassium phosphates, e.g., sodium or potassium phosphate, citrates, e.g., sodium or potassium citrate, maleic acid, acetates, e.g., sodium, potassium or ammonium acetate, tris-(hydroxymethyl)-aminomethane (tris), Tricine, HEPES, MOPS, etc. The amount of buffering agent present in the dried composition may vary. In some instances, the amount is chosen such that upon combination of 1 µg of the dried composition with 10 µl of water, the concentration of the buffer in the resultant aqueous composition ranges from 50 to 500 mM, such as from about 75 to 350 mM, and including from about 225 to 275 mM, e.g., 250 mM. The amount of buffering component in the dried composition may vary, ranging in some instances from 10 to 95, such as from 25 to 90 and including from 50 to 80, e.g., 72.5% wt.%. In certain embodiments the buffering agent will be present in an amount sufficient to provide a pH ranging from about 5.0 to 10.0, such as 6.0 to 9.5 in the resultant aqueous composition prepared from the dried composition upon combination with water.

In some instances, the dried composition includes a cryoprotectant. By cryoprotectant is meant a substance that functions to preserve the components of the composition during the drying, e.g., freeze-drying process. In some instances, cryoprotectants are compounds that do not also function as buffering agents. Suitable cryoprotectants include, but are not limited to: glycols, such as ethylene glycol, propylene glycol, and glycerol, one or more sugars (e.g., one or more disaccharides, such as trehalose, melizitose, raffinose), where the sugars may be mixed with one or more polyalcohols (e.g., mannitol, sorbitol), etc. In those embodiments that include a cryoprotectant, the cryoprotecant may be present in varying amounts, ranging in some instances from 0.01 to 90, such as from 0.05 to 50 and including from 0.1 to 10 wt. %. In some instances, the compositions are cryoprotectant free, in that they do not include a cryoprotectant, at least not in an amount that functions as a cryoprotectant for the components of the compositions. In such instances, the amount of cryoprotectant in the composition, if any, is 1.0 wt.% or less, such as 0.5 wt.% or less, including 0.1 wt.% or less.

In addition to the polymeric transfection agent and buffer, the dried composition may further include a number of different components, such as but not limited to: binders, fillers, bulking agents, tonicity agents, stabilizers, surfactants, solubilizing agents, antioxidants, chelating agents, preservatives and the like. When present, these components may make up 0.1 to 99 wt.%, such as 1 to 50 wt.%, including 2 to 10 wt. %, of the composition. In some instances, the dried composition is made up solely of the transfection agent component and buffer component.

In some instances, the dried compositions include one or more nucleic acids. Nucleic acids of interest include polymers of any length composed of deoxyribonucleotides, ribonucleotides, or combinations thereof, where the length of the nucleic acids may vary, e.g., 10 bases or longer, 20 bases or longer, 50 bases or longer, 100 bases or longer, 500 bases or longer, 1000 bases or longer, 2000 bases or longer, 3000 bases or longer, 4000 bases or longer, 5000 bases or longer or more bases, where an upper limit in certain embodiments is 150,000 bases or less, such as 50,000 bases or less. In certain aspects, the nucleic acid is a polymer composed of deoxyribonucleotides or ribonucleotides or combinations thereof, ranging in length from 10 to 150,000, such as 1,000 to 50,000 and including 2,000 to 15,000 bases.

Nucleic acids of interest include deoxyribonucleic acids (DNA), such as but not limited to: genomic DNA or fragments thereof, complementary DNA (or "cDNA", synthesized from any RNA or DNA of interest), recombinant DNA (e.g., plasmid DNA), etc. Nucleic acids of interest also include ribonucleic acids (RNA), which may be any type of RNA (or sub-type thereof) including, but not limited to, a messenger RNA (mRNA), a microRNA (miRNA), a small interfering RNA (siRNA), a transacting small interfering RNA (ta-siRNA), a natural small interfering RNA (nat-siRNA), a ribosomal RNA (rRNA), a transfer RNA (tRNA), a small nucleolar RNA (snoRNA), a small nuclear RNA (snRNA), a long non-coding RNA (IncRNA), a non-coding RNA (ncRNA), a transfer-messenger RNA (tmRNA), a precursor messenger RNA (pre-mRNA), a small Cajal body-specific RNA (scaRNA), a piwi-interacting RNA (piRNA), an endoribonuclease-prepared siRNA (esiRNA), a small temporal RNA (stRNA), a signal recognition RNA, a telomere RNA, a ribozyme, or any combination of RNA types thereof or subtypes thereof.

When present in the dried compositions, the nucleic acid component may include a single type of nucleic acid (i.e., where all of the nucleic acids have the same length and the same sequence) or the nucleic acid component may include 2 or more, such as 3 or more, including 4 or more, e.g., 5, 6, 7, 8, 9 or 10 or more distinct nucleic acids of at least differing length and/or sequence. The total amount of nucleic acid component in the dried composition may vary, ranging in some instances from 0.001 to 10 wt.%, such as from 0.01 to 5 wt.% and including from 0.01 to 2 wt.%.

In certain instances, at least one of the nucleic acids includes an expression cassette. Expression cassettes of interest include a regulatory sequence operably linked to a coding sequence. Regulatory sequences can include promoters (attached either at the 5' end of the sense strand or at the 3' end of the antisense strand), enhancers, terminators, operators, repressors, and inducers.

A "promoter" is a DNA regulatory region capable of binding RNA polymerase in a cell and initiating transcription of a downstream (3' direction) coding sequence. A promoter may be bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding regions responsible for the binding of RNA polymerase. Eukaryotic promoters will often, but not always, contain "TATA" boxes and "CAT" boxes. Various promoters, including inducible promoters, may be used to drive expression.

The promoters can be regulated or constitutive, and in some instances are regulated (e.g., conditionally active). In some situations it may be desirable to use conditionally active promoters, including, but not limited to, tissue-specific promoters; cell type specific promoters; developmental stage-specific promoters; promoters controlled by the cell cycle; promoters controlled by Circadian rhythm; and promoters whose activity is increased (e.g., activated) or decreased (e.g., suppressed) in response to an external or internal signal.

External and internal signals that affect promoter activity include, but are not limited to, infection of a cell by a microorganism, including, but not limited to, a bacterium (e.g., Mycobacterium spp., Shigella, Chlamydia, and the like), a protozoan (e.g., Trypanosoma spp., Plasmodium spp., Toxoplasma spp., and the like), a fungus, a yeast (e.g., Candida spp.), or a virus (including viruses that infect mammalian cells, such as human immunodeficiency virus, foot and mouth disease virus, Epstein-Barr virus, and the like; viruses that infect plant cells; etc.); change in pH of the medium in which a cell is maintained or a change in internal pH; excessive heat relative to the normal range for the cell or the multicellular organism; excessive cold relative to the normal range for the cell or the multicellular organism; an effector molecule such as a hormone, a cytokine, a chemokine, a neurotransmitter; an ingested or applied drug; a ligand for a cell-surface receptor; a ligand for a receptor that exists internally in a cell, e.g., a nuclear receptor; hypoxia; light; dark; mitogens, including, but not limited to, lipopolysaccharide (LPS), pokeweed mitogen; antigens; sleep pattern; electrical charge; ion concentration of the medium in which a cell is maintained or an internal ion concentration, exemplary ions including sodium ions, potassium ions, chloride ions, calcium ions, and the like; presence or absence of a nutrient; metal ions; a transcription factor; a tumor suppressor; cell-cell contact; and the like.

Cell-cycle controlled promoters include, but are not limited to, an E2F 1 promoter; a cyclin A promoter; a cyclin B promoter; a cyclin C promoter; a cyclin D promoter; a cyclin E promoter; and the like. Cell-cycle controlled promoters are known in the art, and any such promoter can be used. The nucleotide sequences of many such promoters are publicly available, including the following: (GenBank Accession Nos shown in parentheses): human cyclin A1 gene promoter (AF124143); human cyclin B1 gene promoter (U22364; S74452; U36838); mouse cyclin C promoter (U49050); mouse cyclin D1 promoter (AF212040; AF182716); rat cyclin D1 promoter (AF 148946); human cyclin D1 promoter (Z29078); human cyclin D2 promoter (U47284); human cyclin D3 promoter (U47285).

Exemplary tissue-specific or cell type-specific promoters include, but are not limited to, myosin heavy chain promoter for muscle specific expression, Madsen et al. (1998) Circ Res 82(8):908 917; lysosomal acid lipase promoter, Du et al. (1998) Gene 208(2):285 295; pancreatic expression using the amylase promoter, Dematteo et al. (1997) J Surg Res72(2):155 161; cardiac-specific overexpression, Kubota et al. (1997) Circ Res 81(4):627 635; folylpoly-gamma-glutamate synthetase promoter, Freemantle et al. (1997) J Biol Chem 272(40):25373 25379; tissue specific expression using neural restrictive silencer element, Kallunki et al. (1997) J Cell Biol 138(6):1343 1354, placenta specific expression using the HGH promoter, Nogues et al. (1997) Endocrinology 138(8):3222 3227, expression during pregnancy using the prolactin promoter, Schuler et al. (1997) Endocrinology 138(8):3187 3194, tissue specific expression using the alpha1 (VI) collagen promoter, Braghetta et al. (1997) Eur J Biochem 247(1):200 208; B cell specific expression, Lennon et al. (1997) Immunogenetics 45(4):266 273; endothelium specific expression, Ronicke et al. (1996) Circ Res 79(2):277 285, the keratin promoters (e.g., human keratin 14 promoter (Wang et al. 1997 Proc Natl Acad Sci US 94:219 26); bovine cytokeratin gene promoters, BKIII and BKVI (Alexander et al. 1995 Hum Mol Genet 4:993 9); keratin 10 gene promoter (Bailleul et al. 1990 Cell 62:697 708); and tyrosinase promoters (specific for melanocytes)). Epidermal-specific promoters are reviewed in Fuchs et al. 1994 (Princess Takamatsu Symp 24:290 302). Additional cell type-specific and/or tissue-specific promoters include promoters such as albumin (liver specific; Pinkert et al., 1987 Genes Dev. 1:268 277), lymphoid specific promoters (Calame et al., 1988 Adv. Immunol. 43:235 275); in particular promoters of T-cell receptors (Winoto et al., 1989 EMBO J. 8:729 733) and immunoglobulins; Banerji et al., 1983 Cell 33729 740; Queen and Baltimore, ibid. 741 748), neuron-specific promoters (e.g. the neurofilament promoter; Byrne et al., 1989 Proc. Natl. Acad. Sci. USA 86:5473 5477), pancreas-specific promoters (Edlunch et al., 1985 Science 230:912 916) and mammary gland-specific promoters (milk whey promoter, U.S. Pat. No. 4,873,316 and European Application Publication No. 264,166).

Developmentally regulated promoters include, but are not limited to, notch, numb, homeotic genes, murine homeobox promoters (Kessel et al., 1990 Science 249:374 379), and the like.

A promoter, and optionally additional regulatory element(s), is linked to the desired nucleotide sequence using the techniques described above for linkage to vectors. Any techniques known in the art can be used. In other words, the expression vector will provide a transcriptional and translational initiation region, which may be inducible or constitutive, where the coding region is operably linked under the transcriptional control of the transcriptional initiation region, and a transcriptional and translational termination region.

Expression cassettes may have convenient restriction sites located near the promoter sequence to provide for the insertion of nucleic acid sequences encoding heterologous proteins. A selectable marker operative in the expression host may be present. Expression cassettes may be prepared comprising a transcription initiation region, a coding sequence or fragment thereof, and a transcriptional termination region.

As mentioned above, in some instances the expression cassette may be an inducible expression cassette. Inducible expression cassettes of interest include a transcription modulator responsive promoter operably linked to a coding sequence. Transcription modulator responsive promoters of inducible expression cassettes of interest include both a transcription modulator responsive element and a minimal promoter element which are operably linked to each other, where the transcription modulator responsive element may be either upstream or downstream from the minimal promoter element, depending on the particular configuration of the expression cassette. Of interest as transcription modulator responsive elements are prokaryotic operon operator sequences, e.g., tet operator sequences. In some instances, the transcription modulator responsive element comprises at least one tet operator sequence. In one embodiment, a transcription modulator responsive element includes multiple copies (e.g., multimerized or concatemerized copies) of 2 or more tet operator sequences. In one configuration of interest, the tet operator sequence(s) is operatively linked upstream (i.e., 5') of the minimal promoter sequence through a phosphodiester bond at a suitable distance to allow for transcription of the target nucleotide sequence upon binding of a regulatory protein (e.g., the regulatory fusion protein) to the tet operator sequence. While the length of the transcription modulator responsive element may vary, in some instances the length of this element ranges from 5 to 1000 base pairs, such as 10 to 100 base pairs, including 15 to 35 base pairs. Specific tet operator sequences of interest include, but are not limited to, those found in the following tet transcription modulator responsive promoters: the *P*_{TRE} promoter, the *P*_{TRE-Tight} promoter, and the *P*_{TREG3} promoter, all available from Clontech Laboratories, Mountain View, CA.

As summarized above, the transcription modulator responsive promoter further includes a minimal promoter element operably linked to the transcription modulator responsive element. Minimal promoter sequences of interest are sequences which are not themselves transcribed but which serve (at least in part) to position the transcriptional machinery for transcription. The term "minimal promoter" includes partial promoter sequences which define the start site of transcription for a linked coding sequence to be transcribed but which by themselves are not capable of initiating transcription efficiently, if at all. Thus, the activity of such a minimal promoter is dependent upon the binding of a transcription modulator (e.g., a transcription modulator protein as described below) to an operatively linked transcription modulator responsive element (such as one or more tet operator sequences). Minimal promoters of interest include, but are not limited to: the minimal promoter from the human cytomegalovirus (e.g., nucleotide positions between +75 to -53, nucleotide positions between +75 to -31, etc.); the human HSV thymidine kinase promoter; the human U6 promoter and the like. Promoters of interest include those described, e.g., in Gossen, M. and Bujard, H. (1992) Proc. Natl. Acad. Sci. USA 89:5547-5551. While the length of the minimal promoter element may vary, in some instances the length of this element ranges from 25 to 1000, such as 50 to 100 base pairs.

In some instances, the tet responsive element and the minimal promoter may be separated by a linker sequence, which may be any convenient sequence. In such embodiments, the distance between the transcription modulator responsive element (e.g., the tet operator sequence(s)) and the minimal promoter may vary, and in some instances the tet operator sequence(s) is located within 500 base pairs, such as 400 base pairs, including 300 base pairs, e.g., 200 base pairs, upstream of the minimal promoter.

Specific transcription modulator responsive promoter configurations of interest include, but are not limited to: a cytomegalovirus minimal promoter linked to ten tet operator sequences, a herpes simplex virus minimal tk promoter linked to ten tet operator sequences, etc. Specific transcription modulator responsive promoters of interest include those found in the following commercially available products: the *P*_{TRE} promoter, the *P*_{TRE-Tight} promoter, and the *P*_{TREG3} promoter, all available from Clontech Laboratories, Mountain View, CA.

As summarized above, the expression cassette includes a regulatory element, e.g., a promoter, operably linked to a coding sequence. The "coding sequence" of the expression cassette can encode a protein of interest or a nucleic acid of interest (e.g., an inhibitory RNA). Thus, upon induction of transcription of the coding sequence of the expression cassette and translation of the resultant mRNA, in certain embodiments the protein of interest is produced in a host cell or animal. Alternatively, the coding sequence to be transcribed can encode for an active RNA molecule (an RNA molecule that does not encode a protein), e.g., an antisense RNA, an siRNA, an shRNA, a ribozyme, a miRNA, etc. Expression of active RNA molecules in a host cell or animal can be used to regulate functions within the host (e.g., prevent the production of a protein of interest by inhibiting translation of the mRNA encoding the protein, reduce the production of a protein of interest by altering the stability of the encoding mRNA, etc.). While the length of the coding sequence may vary, in some instances the coding sequence has a length ranging from 10 bp to 15,000 bp, such as 50 bp to 5,000 bp and including 100 bp to 1000 bp.

The coding sequence of the expression cassette may be exogenous or endogenous. An "exogenous" coding sequence is a nucleotide sequence which is introduced into the host cell, e.g., into the genome of the host. The exogenous coding sequence may not be present elsewhere in the genome of the host (e.g., a foreign nucleotide sequence) or may be an additional copy of a sequence which is present within the genome of the host but which is integrated at a different site in the genome. An "endogenous" coding sequence is a nucleotide sequence which is present within the genome of the host. An endogenous gene can be operatively linked to an operator sequence(s) to produce an expression cassette of the invention by homologous recombination between an operator sequence recombination vector and sequences of the endogenous gene, such that the native promoter is replaced with the regulatory protein responsive element and the endogenous gene becomes part of an inducible expression cassette.

In some instances, the expression cassette is present on a vector, e.g., a plasmid, an artificial chromosome, e.g. BAC, etc. While the length of the expression cassette and vector on which it is present may vary, in some instances the length ranges from 0.1 kb to 150 kb, such as 1 to 10 kb and including 5 to 10 kb.

In some instances, the expression cassette includes a coding sequence that encodes a viral protein. In such instances, the viral protein may be a component of a viral vector. Viral vectors of interest include, but are not limited to: retroviral, e.g., lentiviral, vectors; adenoviral vectors; adeno-associated virus (AAV) viral vectors, feline immunodeficiency virus (FIV) vectors, rabies virus vectors, avian sarcoma leukosis virus (ASLV) vectors, etc. Expression cassettes of interest in such embodiments may encode one or more viral proteins that can also be provided in trans in a viral packaging cell. Such proteins include, but are not limited to: enzymes, e.g., polymerase, capsid proteins, envelope proteins, regulatory proteins, etc.

In some instances, the nucleic acid component of the dried composition may include one or more expression cassettes encoding one or more viral proteins (e.g., lentiviral proteins, adenoviral proteins, AAV proteins, FIV proteins, human immunodeficiency virus (HIV) proteins, rabies virus proteins, ASLV proteins, Murine Leukemia Virus (MLV), etc.) that are employed in trans for production of vector particles (e.g., lentiviral vector particles), e.g., such as may be present in a packaging cell (e.g., lentiviral packaging cell, e.g., the 293 (HEK293T), HeLa, D17, MDCK, BHK, and Cf2Th packaging cell lines). Viral proteins can be included in any convenient nucleic acid (e.g., linear expression constructs, minicircle or minicircle-like constructs, plasmids, etc.).

The lentiviral genome and the proviral DNA have the three genes found in retroviruses: gag, pol and env, which are flanked by two long terminal repeat (LTR) sequences. The gag gene encodes the internal structural (matrix, capsid and nucleocapsid) proteins; the pol gene encodes the RNA-directed DNA polymerase (reverse transcriptase), a protease and an integrase; and the env gene encodes viral envelope glycoproteins. The 5' and 3' LTR's serve to promote transcription and polyadenylation of the virion RNA's. The LTR contains all other cis-acting sequences necessary for viral replication. Lentiviruses have additional accessory genes including vif, vpr, tat, rev, vpu, nef and vpx.

Retroviral (e.g., lentiviral, HIV, FIV, avian sarcoma leukosis virus (ASLV), Murine Leukemia Virus (MuLV or MLV), and the like) proteins of interest that can be encoded by the expression cassette include, but are not limited to: protease; reverse transcriptase (RT); integrase (IN), e.g., a functional integrase or a non-functional integrase; tat; rev; nef; vpu; vpr; vpx; vif; GAG; GAG-POL (precursor polyprotein); POL (RT and IN); an envelope protein (Env) (e.g., an envelope protein that pseudotypes the virion); as well as fusions, fragments, and/or combinations thereof. Envelope proteins of interest include, but are not limited to: the envelope glycoprotein from the vesicular stomatitis virus (VSV-G); an ecotropic envelope protein such as gp70; RD114; 10A1 (pseudotypes the murine leukemia virus), an amphotropic protein, and the like.

In some cases an envelope protein of a virion determines the viral particle's tropism (i.e., the envelope protein pseudotypes the viral particle). For example, virions pseudotyped with VSV-G can infect both mammalian and non-mammalian cells, while virions pseudotyped with the MLV glycoprotein gp70 (mCAT-1 envelope glycoprotein) can infect mouse and rat cells because gp70 recognizes the mCAT-1 receptor that is expressed primarily on mouse and rat cells.

In cases where an expression cassette encodes a non-functional IN, the sequence encoding the integrase can have a mutation that renders the enzyme defective. As such, transduction of target cells produced with a non-functional IN leads to the formation of circular lentiviral episomes (capable of transient expression of a gene of interest) that do not integrate into the host genome.

Adenoviral proteins and/or nucleic acids of interest that can be encoded by the expression cassette include, but are not limited to: E1 (E1a and/or E1b), E2, E3, E4, and/or VA RNA (e.g., VAI, also known as VA RNAI and/or VAII, also known as VA RNAII, etc.); as well as fusions, fragments, and/or combinations thereof.

Adenoviral associated virus (AAV) proteins and/or nucleic acids of interest that can be encoded by the expression cassette include, but are not limited to: rep (e.g., Rep78, Rep68, Rep52, Rep40), cap (e.g., VP1, VP2, VP3), as well as helper virus proteins including adenovirus E1 (e.g., E1a, E1b), E2, E3, E4, and/or adenovirus VA RNA; as well as fusions, fragments, and/or combinations thereof. The AAV virus particles can require adenovirus helper, or instead can be helper-free.

Rabies virus proteins of interest that can be encoded by the expression cassette include, but are not limited to nucleoprotein (N), phosphoprotein (P), matrix protein (M), glycoprotein (G) and the viral RNA polymerase (L); as well as fusions, fragments, and/or combinations thereof.

Where desired, expression cassettes can be configured to carry the essential sequences for incorporating foreign nucleic acid, for selection and for transfer of the nucleic acid into a host cell. For example, in the retroviral genome, a single nucleic acid molecule that also contains all the necessary cis-acting elements carries all the coding sequences. Biosafety of a vector production system can therefore be achieved by distributing the sequences encoding its various components over independent units to increase the number of crossovers that would be required to re-create a replication competent recombinant.

Multiply attenuated packaging systems (e.g., lentiviral based, e.g., HIV-based, packaging systems) typically comprise only about a third of the viral genes: GAG, encoding the virion main structural proteins, POL, responsible for the retrovirus-specific enzymes, and rev, which encodes a post-transcriptional regulator necessary for efficient gag and pol expression (Dull, et al., 1998). From such an extensively deleted packaging system, the parental virus cannot be reconstituted, since some 60% of its genome has been completely eliminated. In one version of an HIV-based packaging system, Gag/Pol, Rev, VSV-G and the vector are produced from four separate DNA units. Also, the overlap between vector and helper sequences has been reduced to a few tens of nucleotides so that opportunities for homologous recombination are minimized.

Viral particles can be generated by co-expressing the virion packaging elements and the vector genome in a so-called producer cell, e.g., 293T human embryonic kidney cells. These cells may be transiently transfected with a number of nucleic acids. In some cases, from three to four separate nucleic acids (e.g., plasmids) are employed, but the number may be greater depending upon the degree to which the components (e.g., lentiviral components) are broken up into separate units. In some instances, one nucleic acid encodes the core and enzymatic components of the virion (e.g., derived from HIV-1). This nucleic acid can be referred to as the packaging nucleic acid. Another nucleic acid encodes the envelope protein(s) (e.g., VSV-G), and can be referred to as the envelope expression nucleic acid. Yet another nucleic acid encodes the nucleic acid to be transferred to the target cell and can be referred to as the transfer vector.

As has been done with oncoretroviral vectors, some vectors (e.g., some lentiviral vectors) have been made that are "self-inactivating" in that they lose the transcriptional capacity of the viral long terminal repeat (LTR) once transferred to target cells (Zufferey, et al. 1998). This modification further reduces the risk of emergence of replication competent recombinants (RCR) and avoids problems linked to promoter interference. These vectors, or their components, are known as SIN vectors or SIN containing vectors. The SIN design is described in further detail in Zufferey et al., 1998 and U.S. Pat. No. 5,994, 136.

For production of a given viral particle of interest, the dried transfection agent composition may include a nucleic acid that encodes any or all of the proteins and/or nucleic acids (which can be provided in trans) required to produce the desired viral particle. As such, for production of viral particles (e.g., lentiviral particles, adenoviral particles, AAV particles, etc.) a given dried transfection agent composition may include a coding sequence or sequences for any of the viral proteins and/or nucleic acids listed above (e.g., for retroviral, e.g., lentivirus, ASLV, MuLV, HIV, FIV, etc.: protease, RT, IN, gag, pol, env, Tat, Rev, nef, vpu, vpr, vpx, vif, etc.; for adenovirus: E1 (e.g., E1a and/or E1b), E2, E3, E4, VA RNA, etc.; for AAV: rep (e.g., Rep78, Rep68, Rep52, Rep40), cap (e.g., VP1, VP2, VP3), as well as helper virus proteins including adenovirus E1 (e.g., E1a, E1b), E2, E3, E4, and/or adenovirus VA RNAs; for rabies virus: N, P, M, G, L; etc.), where such may be present on the same vector or distributed among two or more, such as three or more, including four or more nucleic acids (e.g., vectors).

As such, a given dried composition may include one or more nucleic acids encoding one or more viral proteins, such as two or more viral proteins, including 3, 4 or 5 or more viral proteins, where the coding sequences for such protein(s) may be present in the same nucleic acid, e.g., nucleic acid vector, or distributed among 2 or more, such as 3 or more, including 4 or more different nucleic acids, e.g., different nucleic acid vectors. In some instances, the nucleic acid component of the dried composition includes coding sequences for all requisite proteins that are needed in trans for producing a viral particle of interest.

In some cases, a nucleic acid encoding one viral protein is represented to a greater or lesser extent relative a nucleic acid encoding another viral protein. As such, the amount of a given viral protein produced (relative to other viral proteins) can be influenced by the relative number of nucleic acid molecules (encoding that protein) that are present in the dried composition. In some cases, a nucleic acid encoding a viral protein (any convenient viral protein) is present in the dried composition at a controlled ratio (e.g., 1.1:1; 1.2:1; 1.3:1; 1.4:1; 1.5:1; 1.6:1; 1.7:1; 1.8:1; 1.9:1; 2:1; 2.25:1; 2.5:1; 2.75:1; 3:1; 3.5:1; 4:1; 4.5:1; 5:1, 6:1; 7:1; 8:1; 9:1; 10:1; etc.) relative to a nucleic acid encoding a different viral protein (any convenient viral protein).

Publications that describe various exemplary viral proteins and/or nucleic acids (in various combinations in various types of expression cassettes) that can be encoded by a subject expression cassette include those described in U.S. patent numbers: (Lentivirus) 5,665,577; 5,981,276; 5,994,516; 5,994,136; 6,979,568; 6,521,457; 6,740,524; 6,013,516; 8,298,826; (Adenovirus) 8,398,968; 8,263,067; 8,163,544; (AAV) 8,163,543; 8,524,678; 7,022,519; 7,229,823; 6,953,690; 6,482,634; (Rabies virus) 7,863,041; and U.S. patent applications: (Lentivirus) 20100137412; 2010022179; 20100098664; 20130096370; (Adenovirus) 20100143304, 20100120155, 20100008889; (AAV) 20130136729.

In some instances, the expression cassette includes a coding sequence that encodes a detectable protein, for example, a fluorescent protein (e.g. a green fluorescent protein, red fluorescent protein, yellow fluorescent protein, cyan fluorescent protein, blue fluorescent protein, and the like); an enzyme that can generate a detectable signal such as fluorescence or luminescence (e.g, luciferase, alkaline phosphatase, secreted alkaline phosphatase, and the like); etc. In some cases, this can facilitate the assessment of transfection efficiency (e.g., via microscopy, FACS, etc.). In some cases, a nucleic acid having an expression cassette where the coding sequence encodes a detectable protein is a component of the lyophilized transfection composition.

The above described dried transfection agent composition may be produced using any convenient protocol. In some instances, an aqueous formulation of the various constituents of the dried composition, e.g., the transfection agent, the buffer, optional cryoprotectant, etc., is first prepared. This aqueous composition can be prepared using any convenient protocol, where the various components may be combined in any convenient or desired order, e.g., sequentially or simultaneously. A representative protocol for preparing this precursor aqueous composition is provided in the Experimental Section, infra. Uncombined water is then separated from the aqueous composition in a manner sufficient to produce the dried composition. Uncombined water may be separated from the composition using any convenient protocol. For example, uncombined water may be separated from the composition by evaporation, lyophilization, etc. For lyophilization protocols, separation of uncombinated water may be achieved using any convenient freeze-dry protocol. In some instances, the temperature of the composition is rapidly reduced, e.g., to flash freeze the device, e.g., through contact with liquid nitrogen etc. The resultant flash frozen composition is then lyophilized using standard protocol, resulting in removal of substantially all free water from the composition present in each well of the device. As substantially all free water is removed from the compositions, the amount of water present in each of the compositions following the lyophilization step may be less than about 0.1 %, such as less than about 0.01% and including less than about 0.001% w/w, such that the compositions are correctly characterized as freeze dried compositions. In some cases, polymer alone or polymer plus nucleic acid is present in liquid form (e.g., in buffer). A 1/10 volume of 3 M sodium acetate (pH 5.5) or ammonium acetate (pH 5.5) can be added, followed by two volumes of ethanol or isopropanol to produce a precipitate that can be pelleted via centrifugation. Vacuum centrifugation may also be used. The subsequent dried sample can be stored at -20C with desiccant.

### METHODS

Aspects of the invention further include methods of introducing a nucleic acid into a cell. By "introducing a nucleic acid into a cell" is meant the transfer of a nucleic acid from a location outside of a cell to a location inside of a cell, such that the nucleic acid is moved from a first location on one side of cell membrane to a second location on the other side of a cell membrane. As such, aspects of the invention include methods of moving a nucleic acid(s) of interest into a target cell from the location outside of the target cell.

Aspects of the methods include producing a liquid transfection composition from dried transfection agent composition, e.g., as described above, a nucleic acid(s) of interest that is to be introduced into the cell. The liquid transfection composition may be an aqueous composition. The nucleic acid of interest present in the liquid transfection composition may vary, ranging in some instances from 1ng to 500 µg, such as 10 ng to 100 µg and including 150 ng to 50 µg. The amount of transfection agent present in the liquid transfection composition may vary, ranging in some instances from 1 ng to 5,000 µg, such as 2 ng to 2,500 µg and including 4 ng to 1,400 µg.

The liquid transfection composition may be produced using any convenient protocol. In some instances, an amount of dried transfection agent composition, e.g., as described above, is combined with a volume of an aqueous liquid that includes the nucleic acid(s) of interest to produce the liquid transfection composition. The weight ratio of solid to liquid that is combined to produce the liquid transfection composition may vary, where in some instances the weight ratio of solid to liquid ranges from 10 to 1 such as 7.5 to 1 and including 5.65 to 1. Combination may be achieved using any convenient protocol, including stirring, agitation, etc., where the combination may occur at a variety of temperatures, where the temperature ranges in some instances from 16 to 30°C, such as 20 to 25°C.

Following production of the liquid transfection composition, the resultant liquid transfection composition is contacted with the target cell under conditions sufficient for the nucleic acid of interest to be introduced into the target cell. In these embodiments, the target cell is contacted with the liquid transfection composition under transfection conditions. In certain instances, an amount of liquid transfection composition that is contacted with an amount of target cells under cell culture conditions and incubated for a sufficient period of time for transduction to occur. In some instances, an amount liquid transfection agent that provides for an amount of nucleic acid ranging from 1 molecule per cell to 1×10E¹² molecules per cell, such as 7×10E⁷ molecules per cell to 7×10E⁸ molecules per cell is contacted with an amount of target cells ranging from 1 to 50 million, such as 10,000 to 100,000 cells. Any convenient culture medium may be employed, where culture media of interest include, but are not limited to: RPMI, DMEM, OptiMEM and the like. The cells and liquid transfection composition may be incubated for varying amounts of time, e.g., from 5 minutes to 5 days, such as 1 to 4 hours, at various temperatures, e.g., ranging from 4 to 42, such as 30 to 37 °C.

The nucleic acid of interest that is introduced into the target cell may vary greatly. Nucleic acids of interest include polymers of any length composed of deoxyribonucleotides, ribonucleotides, or combinations thereof, where the length of the nucleic acids may vary, e.g., 10 bases or longer, 20 bases or longer, 50 bases or longer, 100 bases or longer, 500 bases or longer, 1000 bases or longer, 2000 bases or longer, 3000 bases or longer, 4000 bases or longer, 5000 bases or longer or more bases, where an upper limit in certain embodiments is 150,000 bases or less, such as 50,000 bases or less. In certain aspects, the nucleic acid is a polymer composed of deoxyribonucleotides or ribonucleotides or combinations thereof, ranging in length from 10 to 150,000, such as 1,000 to 50,000 and including 2,000 to 15,000 bases.

Nucleic acids of interest include deoxyribonucleic acids (DNA), such as but not limited to: genomic DNA or fragments thereof, complementary DNA (or "cDNA", synthesized from any RNA or DNA of interest), recombinant DNA (e.g., plasmid DNA), etc. Nucleic acids of interest also include ribonucleic acids (RNA), which may be any type of RNA (or sub-type thereof) including, but not limited to, a messenger RNA (mRNA), a microRNA (miRNA), a small interfering RNA (siRNA), a transacting small interfering RNA (ta-siRNA), a natural small interfering RNA (nat-siRNA), a ribosomal RNA (rRNA), a transfer RNA (tRNA), a small nucleolar RNA (snoRNA), a small nuclear RNA (snRNA), a long non-coding RNA (IncRNA), a non-coding RNA (ncRNA), a transfer-messenger RNA (tmRNA), a precursor messenger RNA (pre-mRNA), a small Cajal body-specific RNA (scaRNA), a piwi-interacting RNA (piRNA), an endoribonuclease-prepared siRNA (esiRNA), a small temporal RNA (stRNA), a signal recognition RNA, a telomere RNA, a ribozyme, or any combination of RNA types thereof or subtypes thereof.

The nucleic acid of interest may include an expression cassette, e.g., as described above. As such, the nucleic acids may include a regulatory sequence operably linked to a coding sequence. Regulatory sequences can include promoters (attached either at the 5' end of the sense strand or at the 3' end of the antisense strand), enhancers, terminators, operators, repressors, and inducers, e.g., as described above. In some instances, the expression cassette is present on a vector, e.g., a plasmid, an artificial chromosome, e.g. BAC, etc. While the length of the expression cassette and vector on which it is present may vary, in some instances the length ranges from 0.1 kb to 150 kb, such as 1 to 10 kb and including 5 to 10 kb.

The coding sequence of the expression cassette may be exogenous or endogenous. An "exogenous" coding sequence is a nucleotide sequence which is introduced into the host cell, e.g., into the nucleus of the host cell, into the genome of the host cell, etc. The exogenous coding sequence may not be present elsewhere in the genome of the host (e.g., a foreign nucleotide sequence), may be an additional copy of a sequence which is present within the genome of the host but which is integrated at a different site in the genome, or may be a variant (e.g., containing a mutation, e.g., an insertion, a deletion, a substitution, etc.; encoding a fusion protein; and the like) of a sequence which is present within the genome of the host. An "endogenous" coding sequence is a nucleotide sequence which is present within the genome of the host. An endogenous gene can be operatively linked to an operator sequence(s) to produce an expression cassette of the invention by homologous recombination between an operator sequence recombination vector and sequences of the endogenous gene, such that the native promoter is replaced with the regulatory protein responsive element and the endogenous gene becomes part of an inducible expression cassette.

In some instances, the nucleic acid of interest comprises a nucleic acid that is to be packaged into a viral particle. In such instances, the nucleic acid may include a packaging sequence and an expression cassette that includes a coding sequence of interest. By packaging sequence (e.g., a Psi site, a Psi sequence) is meant a sequence used for efficient encapsidation of nucleic acid into viral particles. Thus, when the nucleic acid of interest includes a packaging sequence and an expression cassette that includes a coding sequence of interest, the nucleic acid of interest will be packaged into the produced viral particles. For example, if a packaging sequence is missing from a lenti viral genome, the cis defect prevents encapsidation of genomic RNA. However, the resulting mutant remains capable of directing the synthesis of all virion proteins.

In some instances, the nucleic acid of interest that is combined with the dried transfection agent in order to produce the liquid transfection composition includes a vector domain that is selected from a viral genome of a virus selected from the group of retroviral, adenoviral, adeno-associated, vaccinia, herpes, foamy, etc. viruses, where such viruses are commonly used for gene transfer applications. In some instances, the vector domain is a retroviral vector region, such that it is a domain derived from a retrovirus. Retroviruses are any virus belonging to the family Retroviridae, comprising single-stranded RNA animal viruses characterized by two unique features. First, the genome of a retrovirus is diploid, consisting of two copies of the RNA. Second, this RNA is transcribed by the virion-associated enzyme reverse transcriptase into double-stranded DNA. This double-stranded DNA or provirus can then integrate into the host genome and be passed from parent cell to progeny cells as a stably-integrated component of the host genome. In some instances, the retroviral vector region is a lentiviral vector region, e.g., a vector derived from a lentivirus. Lentiviruses are members of the retrovirus family. Lentivirus vectors may be pseudotyped with VSV-G, and have been derived from the human immunodeficiency virus (HIV), the etiologic agent of the human acquired immunodeficiency syndrome (AIDS); visna-maedi, which causes encephalitis (visna) or pneumonia in sheep; the caprine arthritis-encephalitis virus, which causes immune deficiency, arthritis, and encephalopathy in goats; equine infectious anemia virus (EIAV), which causes autoimmune hemolytic anemia and encephalopathy in horses; feline immunodeficiency virus (FIV), which causes immune deficiency in cats; bovine immune deficiency virus (BIV) which causes lymphadenopathy and lymphocytosis in cattle; and simian immunodeficiency virus (SIV), which causes immune deficiency and encephalopathy in non-human primates. Vectors that are based on HIV may retain <5% of the parental genome, and <25% of the genome may be incorporated into packaging constructs, which minimizes the possibility of the generation of revertant replication-competent HIV. The vector region may include sequences form the 5' and 3' LTRs of a lentivirus. In some instances, the vector domain includes the R and U5 sequences from the 5' LTR of a lentivirus and an inactivated or self-inactivating 3' LTR from a lentivirus.

The term "long terminal repeat" or "LTR" refers to the sequences of DNA that repeat and are found at either end of proviral DNA formed by reverse transcription of retroviral RNA. LTRs are used by viruses to insert their genetic material into host genomes (e.g., the ends of the LTRs participate in integration of the provirus into the host genome). The multistep process of reverse transcription results in the placement of LTRs at either end of the proviral DNA. Once the provirus has been integrated into the host genome, the LTR on the 5' end usually serves as the promoter for the retroviral genome.

The LTR sequences may be LTR sequences from any lentivirus from any species. For example, they may be LTR sequences from HIV, SIV, FIV or BIV. Where desired, the effector library may be made up of self-inactivating vectors that contain deletions of the regulatory elements in the downstream long-terminal-repeat sequence, eliminating transcription of the packaging signal that is required for vector mobilization. As such, the vector region may include an inactivated or self-inactivating 3' LTR. The 3' LTR may be made self-inactivating by any convenient method. For example, the U3 element of the 3' LTR may contain a deletion of its enhancer sequence, such as the TATA box, Sp1 and NF-kappa B sites. As a result of the self-inactivating 3' LTR, the provirus that is integrated into the host cell genome will comprise an inactivated 5' LTR. Optionally, the U3 sequence from the lentiviral 5' LTR may be replaced with a promoter sequence in the viral construct. This may increase the titer of virus recovered from the packaging cell line. An enhancer sequence may also be included.

Of interest in such embodiments are methods where a nucleic acid to be packaged (e.g., a nucleic acid having a packaging sequence, described above) is combined with a dried transfection agent composition that further includes one or more expression cassettes encoding one or more of, including all of, the viral proteins that are to provided in trans to produce a viral particle, e.g., as described above.

In such embodiments, the nucleic acid to be packaged is in liquid form (e.g., dissolved in water or buffer) when it contacts the dried transfection agent composition, thus producing a liquid transfection agent, which includes the nucleic acid to be packaged in a viral particle, and optionally one or more of, including all of, the coding sequences to produce the requisite viral proteins in trans. The liquid transfection agent is contacted with a target cell and maintained under viral particle production conditions. The target cell may or may not include one or more coding sequences for viral proteins to be provided in trans, such that it may or may not be a packaging cell.

As such, in some instances the liquid transfection composition produced from the dried transfection agent composition may then be used to transfect a suitable target cell line, e.g., packaging cell line, for production of desired viral particles. Where the cell line is one that includes one or more coding sequences for viral proteins to be provided in trans, the target cell line may be a packaging cell line. The packaging cell line provides one or more of, including all of, the viral proteins that are required in trans for the packaging of the viral genomic RNA into viral particles. The packaging cell line may be any cell line that is capable of expressing the viral proteins, including 293 (e,g., HEK293T), HeLa, D17, MDCK, BHK, and Cf2Th. In some embodiments, the effector construct is used together with a viral reporter construct which may comprise one or more reporter genes under the control of a constitutive or conditional (regulatable) promoter. In one embodiment, at least one of the reporter genes is controlled by a signaling pathway-specific promoter (conditional) and a second reporter gene is controlled by a constitutive promoter. The packaging cell line may stably express necessary viral proteins. Such a packaging cell line is described, for example, in U.S. Pat. No. 6,218,181. Alternatively, a packaging cell line may be transiently transfected with plasmids comprising nucleic acids that encode the necessary viral proteins. In another embodiment, a packaging cell line that does not stably express the necessary viral proteins is co-transfected with two or more plasmids. One of the plasmids comprises the viral construct comprising the coding sequence of interest. The other plasmid(s) comprises nucleic acids encoding the proteins necessary to allow the cells to produce functional virus that is able to infect the desired host cell. The packaging cell line may not express envelope gene products. In this case, the packaging cell line will package the viral genome into particles that lack an envelope protein. As the envelope protein is responsible, in part, for the host range of the viral particles, the viruses preferably are pseudotyped. A "pseudotyped" retrovirus is a retroviral particle having an envelope protein that is from a virus other than the virus from which the RNA genome is derived. The envelope protein may be from a different retrovirus or a non-retrovirus. One envelope protein is the vesicular stomatitius virus G (VSV-G) protein. Thus, the packaging cell line may be transfected with a plasmid comprising sequences encoding a membrane-associated protein, such as VSV-G, that will permit entry of the virus into a host cell. One with skill in the art can choose an appropriate pseudo type for the host cell used. In addition to conferring a specific host range, a chosen pseudo type may permit the virus to be concentrated to a very high titer. Viruses alternatively can be pseudotyped with ecotropic envelope proteins that limit infection to a specific species.

In some embodiments, the method further includes obtaining, e.g., by separating, the produced viral particles from the cell. Any convenient separation protocol may be employed. Examples of suitable separation protocols include, but are not limited to: filtration, centrifugation, precipitation, surface and/or antibody based capture and the like. In some cases, viral particles are harvested from the cell supernatant post-transfection. In certain cases, the viral particles may be isolated (i.e., harvested) by brief centrifugation (e.g., 500xg for 10 minutes) to remove cellular debris. This can be optionally followed by filtration (for example on 0.45 um pore filters, e.g., cellulose acetate filters, polysulfonic (low protein binding) filters, PVDF filters, etc.). Harvested viral particles may be stored at 4 °C for short periods (hours to days), but should be frozen at -20°C or -80°C for long-term storage.

### KITS

Aspects of the present disclosure also include kits. The kits may include, e.g., one or more dried transfection agent compositions, e.g., as described above. In some instances, the kits may further include a vector comprising a pro-expression cassette which includes a cloning site operably connected to a regulatory element, e.g., a promoter, which is configured to receive a coding sequence of interest. In some instances, this pro-expression cassette is a viral vector domain, e.g., as described above, such that it may include a packaging sequence, as well other desired viral genomic sequences, e.g., as described above.

Kits can include lyophilized transfection compositions, e.g., as described above, that are distributed in multi-well substrates (e.g., which can facilitate high-throughput applications), or analogous structures that include a plurality of liquid containment means, where the substrates include in at least one or more of their wells at least one lyophilized transfection reagent. The subject substrates can have a plurality of reaction chambers or wells (e.g., at least 2, at least 6, at least 24, at least 96, at least 384, at least 1536, etc.). For example, the substrate can be a 2-well plate, a 6-well plate, a 24-well plate, a 96-well plate, a 384-well plate, a 1536-well plate, etc. The wells or reaction chambers may be integrated into a base holder (and therefore not removable or separable therefrom) or exist as separate containment structures, e.g., as described below. An embodiment of a substrate is a PCR plate, such as a multi-well PCR plate, e.g., as are currently commercially available. The overall size and configuration of the substrate will be one that provides for simple, manual or automated handling, where the substrate may be disc shaped, slide shaped, rectangular, and the like. The length of the substrate can range from 10 to 1000 mm, (e.g., from 20 to 200 mm, from 100 to 150 mm, etc.). The height of the substrate can range from 1 to 50 mm (e.g., from 5 to 30 mm, from 10 to 25 mm, etc.). The width of the substrate can range from 10 to 1000 mm (e.g., from 20 to 200 mm, from 50 to 150 mm, etc.). Each reaction chamber of the substrate is a container having an open top and at least one wall configured in a manner sufficient to form a fluid container, where the number of distinct walls depends on the cross-sectional shape of the container, e.g. 1 wall for a container having a circular cross-sectional shape and 4 walls for a container having a square cross-sectional shape. The structure of the well can be any convenient shape, e.g., conical, such that the wall(s) gradually merge to a point at the bottom of the well. However, other configurations are also encompassed, such as wells that have a distinct bottom surface, e.g., a planar bottom surface. The reaction chamber may have a variety of cross-sectional shapes, including circular, triangular, rectangular, square, pentagon, hexagon, etc., including irregular, and in many embodiments has a circular, rectangular or square cross-sectional shape. Therefore, the number of distinct walls of the well is at least one, and can be 2, 3, 4, 5, 6 or more, depending on the cross-sectional shape.

The volume of fluid capable of being contained in the reaction chambers or wells can range from about 0.0001 to 10 ml (e.g., from 0.001 to 1 ml, from 0.005 to 0.1 ml, etc.). The height of the walls will generally be uniform and will be sufficient to form a reaction chamber that is capable of holding a desired amount of fluid, e.g. transfection reagent composition in liquid form (e.g., after liquid is added to the lyophilized transfection reagent composition). As such, the height of each wall of the reaction chamber will be at 0.1 mm or more (e.g., 1 mm or more, 10 mm or more, 20mm or more, 50mm or more, 100mm or more, etc.).

The material from which the multi-well substrates are fabricated will can be a rigid material capable of providing a physical support and structure to the substrate, where suitable materials include, but are not limited to: glass (glasses or silicon), plastics, e.g., polytetrafluoroethylene, polyvinylidenedifluoride, polypropylene, polystyrene, polycarbonate, combinations or modifications thereof (e.g., heat stable materials), and the like, metals, e.g. aluminum, gold, titanium, or alloy and the like, where in many embodiments the material can be transparent, at least to certain light wavelengths. The walls and the bottom surface of the reaction chambers making up the substrate may be fabricated from the same or different materials.

The multi-well substrates may be fabricated using any convenient methodology. Depending on the nature of the material from which the substrate is fabricated, fabrication may involve injection molding, micro machining or other suitable fabrication techniques. The substrate may be prepared as a single unit, or the bottom portion of the substrate may be fabricated separately from the walls, where the substrate is then assembled by placing the walls on the surface of the plate in a stable relationship.

In some embodiments, the wells of a multi-well substrate can be sealed such that the lyophilized composition of each well does not come into contact with the ambient environment of the substrate prior to use. Any convenient sealing means may be present in the substrates, so long as the sealing process and procedure for using the same does not adversely impact the nature of the lyophilized composition. The sealing procedure (and seal) should be substantially, if not completely, water vapor proof such that water vapor cannot pass from the outside environment through the seal into the well. Because of the sealing, a desiccant material is not present in the wells and is not required to maintain the activity of the lyophilized composition. An exemplary seal, a removable component, e.g., a removable cap, a heat seal, a multi-well seal component, e.g., cap, which is a single component sealing two or more distinct wells, etc.

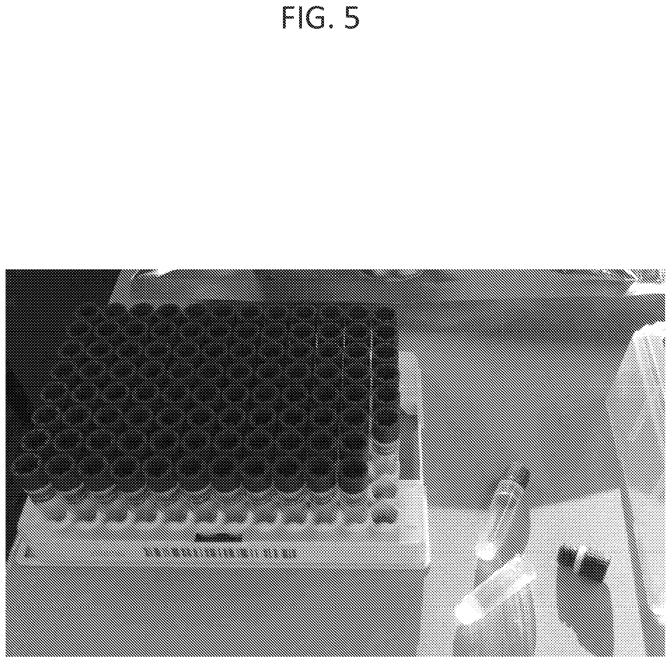

FIG. 5 provides of view of a multi-well substrate according to one embodiment of the invention. In FIG. 5, a multi-well substrate includes a holder configured to hold a plurality of individual, removable containers (e.g., reaction chambers or wells), each of which is filled with a lyophilized composition in accordance with the invention. Each of the containers includes an individual removable cap structure which seals the container. As such, in this embodiments the multi-well substrate includes a base holder configured to hold a plurality of removable containers which are not integrated with the base holder, where the removable containers are, in this embodiment, vials that are sealable with a removable cap structure.

The subject kits, components and methods facilitate automation of the packaging process for high throughput packaging of nucleic acid libraries/collections. For example, lyophilized transfection reagent compositions can be present in multi-well format (as described above, e.g., 386-well, 1536-well, etc.), and libraries of nucleic acids (nucleic acids to be delivered to target cells) in liquid form can be added to the wells in liquid form to produce liquid transfection reagent compositions. Alternatively, nucleic acids to be delivered to target cells can be mixed into the transfection reagent composition prior to lyophilizing. In some such cases, water can be added to the wells to produce the transfection reagent compositions in liquid form.

The transfection reagent compositions (e.g., in multi-well format) can then be contacted with target cells (e.g., still in multi-well format). If the transfection reagent compositions include viral proteins (e.g., in order to produce virus particles), the target cells can be packaging cells. The produced virus particles can then be collected (i.e., harvested) (e.g., still in multi-well format).

Thus, the process of producing a transfection reagent composition with a nucleic acid to be delivered (as well as the optional process of packaging the nucleic acids into viral particles) can be readily automated for high-throughput production.

Components of the kits may be present in separate containers, or multiple components may be present in a single container. For example, the dried nucleic acid composition and the pro-expression cassette may be present in different containers, or combined in a single container, as desired. In some instances, the container(s) are sterile packaging containers. All of the components may be including a single packaging element, e.g., box, tray, bag, such as for ease of handling.

In addition to the above-mentioned components, a subject kit may further include instructions for using the components of the kit, e.g., to practice the subject methods. The instructions are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g., portable flash drive, CD-ROM, diskette, Hard Disk Drive (HDD) etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### UTILITY

The subject methods and compositions find use in a variety of research an clinical applications. The methods and compositions can be used for direct delivery of nucleic acid into cells (e.g., lyophilized reagent plus nucleic acid to be delivered); and/or indirect delivery of nucleic acid to cells (e.g., lyophilized reagent, plus nucleic acid to be delivered, plus nucleic acid(s) encoding viral proteins and/or viral genomes). In cases of indirect delivery, the nucleic acid of interest is delivered to target cells after being packaged into a viral particle (e.g., retroviral, lentiviral, adenoviral, AAV, etc.). In some cases, the nucleic acid(s) encoding the viral component(s) is present in liquid form and not part of the lyophilized transfection composition. In some cases, at least one nucleic acid encoding at least one viral component (and in some cases all viral components) are encoded by nucleic acid(s) that are included in the lyophilized transfection composition.

Delivery of nucleic acids into cells can be used for *in vitro* or *in vivo* expression of any desired protein and/or peptide, as well as any desired non-coding RNA (e.g., miRNA, siRNA, shRNA, etc.). Thus, nucleic acid delivery can be used to increase or decrease the function of any desired gene/protein in any desired cell type from any desired species (e.g., mammal, insect, fish, etc.), in any desired context (e.g., tissue slices, cell culture, 3D cell culture, *in vivo,* etc.).

Delivery of nucleic acids to cells is useful for a wide variety of research and clinical uses. For example, the delivery of nucleic acids to individuals *in vivo* (sometimes referred to in the at as "gene therapy") can be used to treat almost any disease and/or condition (e.g., cancer, diabetes, liver damage/failure, kidney damage and/or failure, pancreatic damage and/or failure, digestive conditions, hematopoietic conditions, etc.). For *ex vivo* gene therapy, nucleic acids can be introduced into cells, and the cells can be transplanted into a subject.

Any type of nucleic acid can be delivered to a target cell using the subject methods and/or compositions. A list of exemplary nucleic acids includes, but is not limited to: nucleic acids encoding genome modification proteins (DNA modifying enzymes) (e.g., CRE recombinase, meganucleases, zinc-finger nucleases, Cas-9 nuclease, TAL effector nucleases, and the like); RNA and/or protein modifying enzymes (e.g., Csy4 nuclease); Fluorescent proteins; Cell cycle control proteins; kinases; structural proteins; signaling proteins; secreted proteins; receptor proteins; apoptotic proteins; translation regulators; peptide antigens; cell programming factors (e.g., Oct4/Sox2/Klf4/Myc); cell differentiation factors (e.g., miRNAs, transcription factors, etc.); nuclear proteins (e.g., transcription factors, cell cycle proteins); cytosolic proteins (e.g., peptide modifying enzymes, apoptotic factors, fluorescent proteins, kinases); and proteins important for vaccine development; as well as nucleic acids encoding non-coding RNAs (e.g., miRNAs, siRNAs, shRNAs) that regulate any desired target protein (e.g., and of the above proteins).

The following examples are offered by way of illustration and not by way of limitation.

### EXPERIMENTAL

### Example 1

To simplify the process utilized by standard transfection protocols, appropriately dispersed transfection reagent was prepared and the resultant mixture was lyophilized. This dried down reagent could then be used directly for transfection by simply adding a solution of DNA and reconstituting the lyophilized powder.

XFECT^{™} polymer was prepared as a solution in DMSO. The XFECT^{™} transfection buffer normally supplied with the reagent in liquid form is a 25 mM sodium acetate buffer at pH 5.0. In some cases, a more concentrated 250mM sodium acetate buffer was used for lyophilization.

For transfection in a single well of a 6-well plate, the lyophilization mix was prepared as follows: 1.5 µl of XFECT^{™} polymer was added to 1.66 µl of 3M NaOAc (sodium acetate), pH 5.0 and 16.84 µl water, and mixed by vortexing. This mixture was then dried in a lyophilizer. For use in transfection, 5 ug DNA was dissolved in 100µl water and added to the dried lyophilization reagent (XFECT^{™} single shots) and mixed by vortexing on high till the solution was clear and homogeneous. The resulting solution was allowed to incubate for 10 min at room temperature in order to form nanoparticles and then was added to cells. Transfection efficiency and toxicity profiles for the lyophilized version of the transfection reagent were comparable to the transfection efficiency and toxicity profiles for the liquid version of the transfection reagent.

### Example 2

Various lyophilized mixtures were tested for their ability to transfect HEK293T cells (293T cells)(packaging cells) and for their ability to induce the HEK293T cells to produce virus.

The tested lyophilized mixtures included:
(3) lyophilized XFECT^{™} transfection reagent (added to liquid LENTI-X^{™} packaging mix and a liquid transfer vector expressing ZsGreen1 fluorescent protein (LXZsG));
(2) lyophilized XFECT^{™} reagent with lyophilized LENTI-X^{™} packaging mix (added to liquid LXZsG); and
(1) lyophilized XFECT^{™} with lyophilized LENTI-X^{™} packaging mix and with lyophilized LXZsG.

The above-lyophilized formulations were tested against a control of:
(4) liquid version of XFECT^{™} (added to liquid LENTI-X^{™} packaging mix and liquid LXZsG).

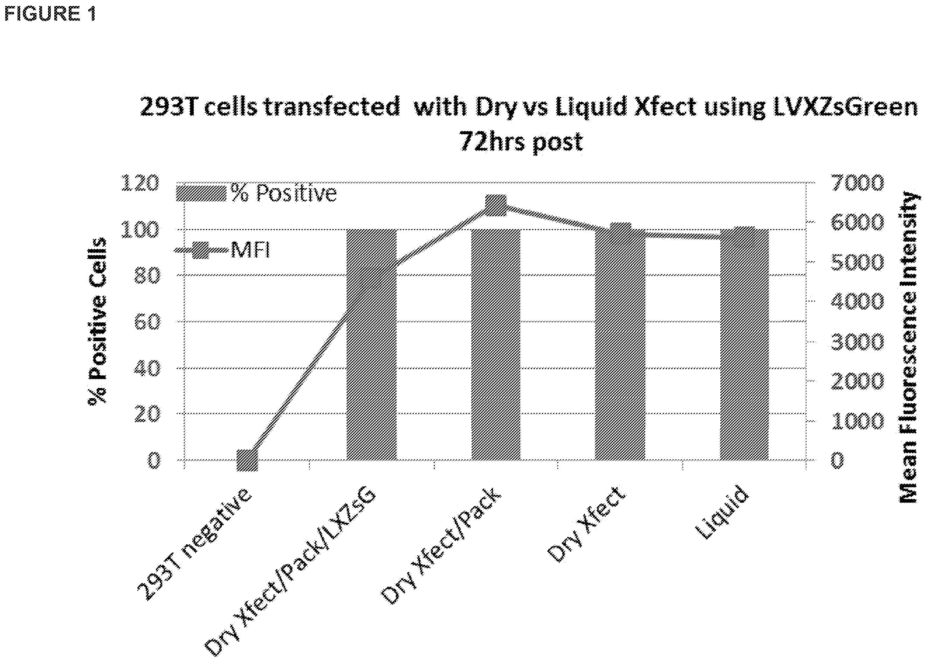

Thus, all 4 conditions contained (i) XFECT^{™} transfection reagent, (ii) LENTI-X^{™} packaging mix, and (iii) LXZsG. The labels on the X-axis for Figures 1-3 refer to the components that where lyophilized prior to transfection ("liquid" indicates that none of the components were lyophilized).

As shown in **FIG. 1****,** HEK293T cells were transfected with the above mixtures and fluorescence activated cell sorting (FACS) analysis of transfected cells demonstrated that all tested mixtures of the transfection reagent transfected at high efficiency. Only slight variations in the mean fluorescent intensity were observed. Thus, the lyophilized formulations transfected cells at a similar level of efficiency compared to a fresh liquid formulation.

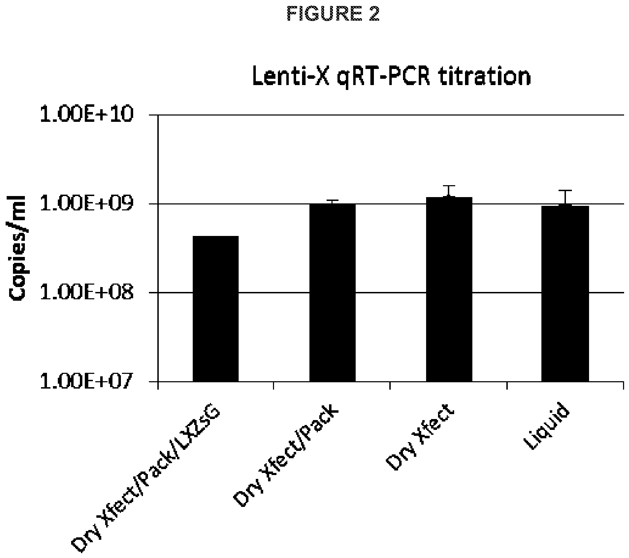

As shown in **FIG. 2****,** Virus-containing supernatants were collected from the transfected 293T cells and the supernatant was subjected to the LENTI-X^{™} qRT-PCR titration Kit (cat# 631235) which is a qRT-PCR assay where a specific portion of the lentiviral RNA genome is amplified and measured against a template of known quantity. All lyophilized packaging formulations produced significant amounts of lentivirus comparable to that of the liquid formulation **(****FIG.2****).**

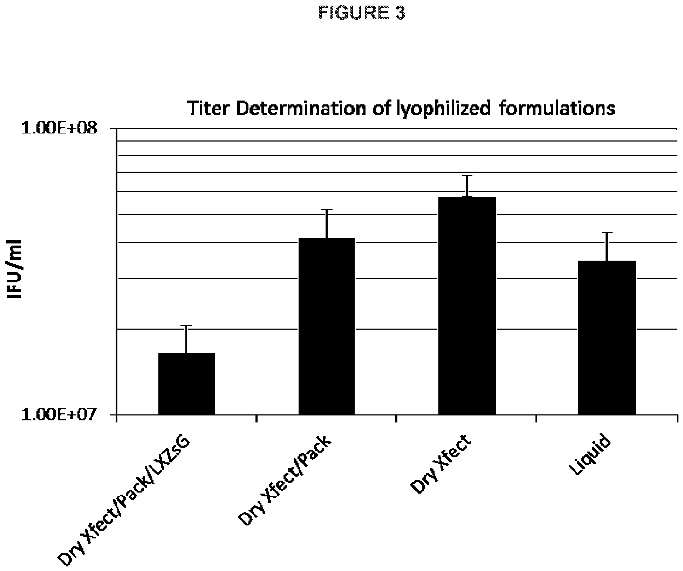

As shown in **FIG. 3****,** to test for the ability of viruses produced by using the lyophilized packaging formulations to infect cells, virus-containing supernatants were collected from the transfected 293T cells and the supernatants were exposed to HT1080 cells. Functional viral titer was then determined **(****FIG.3****).** Briefly, HT1080 cells were plated and transduced with a serial dilution of the virus-containing supernatants. After 48 hours, the transduced cells were analyzed for green fluorescence by FACS and the percentage of positive cells determined. To ensure one copy of virus per cell, only cultures with less than 20% positive cells were used for infectious unit per ml (IFU/ml) calculations. The lyophilized packaging formulations produced significant amounts of lentivirus with 20% and 67% more IFUs being produced in the Dry XFECT^{™} + Packaging DNA and Dry XFECT^{™} no DNA respectively.

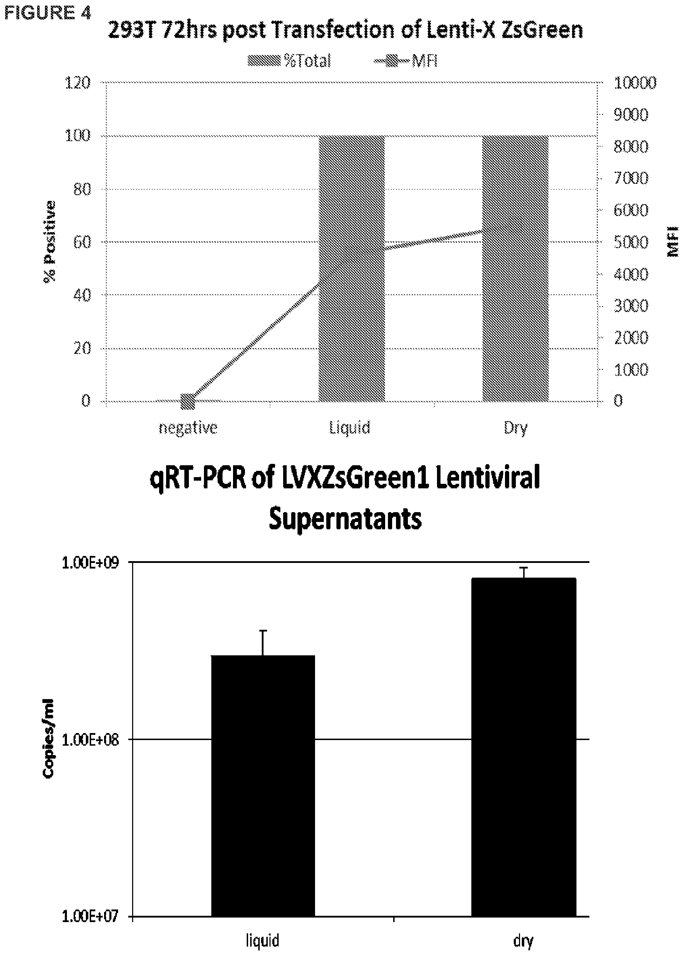

As shown in **FIG. 4****,** additional samples (N=8 liquid; N=13 dry) were tested to confirm the performance of the lyophilized transfection-retroviral packaging mix. Transfection efficiency was determined by FACS analysis (**FIG.4****,** top panel: % positive cells and mean fluorescence intensity (MFI)) and copies/ml of virus was determined by qRT-PCR (**FIG.4****,** bottom panel) as described above. The lyophilized packaging mix transfected at an efficiency similar to the liquid formulation but produced a higher output of viral particles as measured by qRT-PCR.

## Claims

1. A dry composition comprising:
a poly(β-amino ester) polymeric transfection agent;
nucleic acids encoding all requisite proteins for producing a retroviral particle, wherein the nucleic acids are present on different vectors; and
a buffer selected from the group consisting of a potassium phosphate buffer, a citrate buffer, a maleic acid buffer, an acetate buffer, a tris-(hydroxymethyl)-aminomethane (tris) buffer, a tricine buffer, a HEPES buffer and a MOPS buffer.

2. The dry composition according to claim 1, wherein the dry composition is a lyophilized composition.

3. The dry composition according to claim 1 or claim 2, wherein the composition is cryoprotectant free.

4. The composition according to any one of claims 1-3, wherein the nucleic acids each comprise an expression cassette encoding one or more retroviral protein(s), and wherein the proteins for producing a retroviral particle comprise GAG-POL or VSG-G.

5. The composition according to any one of claims 1 to 4, wherein the nucleic acids encoding all requisite proteins for producing a retroviral particle are distributed among two or more, three or more or four or more vectors.

6. The composition according to any one of claims 1-5, wherein the retroviral particle is a lentiviral particle.

7. A method of introducing a nucleic acid of interest into a cell *in vitro,* the method comprising:
a) combining a composition according to any one of claims 1 to 6 with a solvent suitable for introducing a nucleic acid into a cell and comprising the nucleic acid of interest to produce a liquid transfection solution; and
b) contacting the liquid transfection solution with the cell *in vitro* to introduce the nucleic acid into the cell.

8. The method according to claim 7, wherein the concentration of the buffer in the liquid transfection solution is in a range from 50 mM to 500 mM.

9. The method according to claim 7 or claim 8, wherein the nucleic acid of interest encodes a mRNA, a protein or an active RNA molecule.

10. A kit comprising:
a container comprising a plurality of individual reaction chambers, where each of the reaction chambers comprises a dry composition comprising:
a poly(β-amino ester) polymeric transfection agent;
nucleic acids encoding all requisite proteins for producing a retroviral particle, wherein the nucleic acids are present on different vectors; and
a buffer selected from the group consisting of a potassium phosphate buffer, a citrate buffer, a maleic acid buffer, an acetate buffer, a tris-(hydroxymethyl)-aminomethane (tris) buffer, a tricine buffer, a HEPES buffer and a MOPS buffer.

11. The kit according to claim 10, further comprising a vector comprising a pro-expression cassette which includes a cloning site operably connected to a regulatory element.

12. The method according to claim 7, wherein the solvent comprises an aqueous liquid.

13. The kit according to claims 11 or 12, wherein the retroviral particle is a lentiviral particle.

## Patentansprüche

1. Trockene Zusammensetzung, umfassend:
ein polymeres Poly(β-aminoester)-Transfektionsmittel;
Nukleinsäuren, die alle erforderlichen Proteine zur Herstellung eines retroviralen Partikels codieren, wobei die Nukleinsäuren auf verschiedenen Vektoren vorhanden sind; und
einen Puffer, ausgewählt aus der Gruppe bestehend aus einem Kaliumphosphatpuffer, einem Citratpuffer, einem Maleinsäurepuffer, einem Acetatpuffer, einem Tris(hydroxymethyl)aminomethan (Tris)-Puffer, einem Tricinpuffer, einem HEPES-Puffer und einem MOPS-Puffer.

2. Trockene Zusammensetzung nach Anspruch 1, wobei die trockene Zusammensetzung eine lyophilisierte trockene Zusammensetzung ist.

3. Trockene Zusammensetzung nach Anspruch 1 oder Anspruch 2, wobei die Zusammensetzung frei von Kryoprotektiva ist.

4. Zusammensetzung nach einem der Ansprüche 1-3, wobei die Nukleinsäuren jeweils eine Expressionskassette umfassen, die ein oder mehrere retrovirale Proteine codiert, und wobei die Proteine zur Herstellung eines retroviralen Partikels GAG-POL oder VSG-G umfassen.

5. Zusammensetzung nach einem der Ansprüche 1 bis 4, wobei die Nukleinsäuren, die alle erforderlichen Proteine zur Herstellung eines retroviralen Partikels codieren, auf zwei oder mehr, drei oder mehr oder vier oder mehr Vektoren verteilt sind.

6. Zusammensetzung nach einem der Ansprüche 1-5, wobei es sich bei dem retroviralen Partikel um ein lentivirales Partikel handelt.

7. Verfahren zum Einbringen einer interessierenden Nukleinsäure in eine Zelle *in vitro,* wobei das Verfahren umfasst:
a) Kombinieren einer Zusammensetzung nach einem der Ansprüche 1 bis 6 mit einem Lösungsmittel, das zum Einbringen einer Nukleinsäure in eine Zelle geeignet ist und die interessierende Nukleinsäure umfasst, um eine flüssige Transfektionslösung herzustellen; und
b) Inkontaktbringen der flüssigen Transfektionslösung mit der Zelle *in vitro,* um die Nukleinsäure in die Zelle einzubringen.

8. Verfahren nach Anspruch 7, wobei die Konzentration des Puffers in der flüssigen Transfektionslösung in einem Bereich von 50 mM bis 500 mM liegt.

9. Verfahren nach Anspruch 7 oder Anspruch 8, wobei die interessierende Nukleinsäure eine mRNA, ein Protein oder ein aktives RNA-Molekül codiert.

10. Kit, umfassend:
einen Behälter, der eine Vielzahl einzelner Reaktionskammern umfasst, wobei jede der Reaktionskammern eine trockene Zusammensetzung umfasst,
die Folgendes umfasst:
ein polymeres Poly(β-aminoester)-Transfektionsmittel;
Nukleinsäuren, die alle erforderlichen Proteine zur Herstellung eines retroviralen Partikels codieren, wobei die Nukleinsäuren auf verschiedenen Vektoren vorhanden sind; und
einen Puffer, ausgewählt aus der Gruppe bestehend aus einem Kaliumphosphatpuffer, einem Citratpuffer, einem Maleinsäurepuffer, einem Acetatpuffer, einem Tris(hydroxymethyl)aminomethan (Tris)-Puffer, einem Tricinpuffer, einem HEPES-Puffer und einem MOPS-Puffer.

11. Kit nach Anspruch 10, ferner umfassend einen Vektor, umfassend eine Pro-Expressionskassette, die eine Klonierungsstelle, funktionsfähig verknüpft mit einem regulatorischen Element, enthält.

12. Verfahren nach Anspruch 7, wobei das Lösungsmittel eine wässrige Flüssigkeit umfasst.

13. Kit nach den Ansprüchen 11 oder 12, wobei es sich bei dem retroviralen Partikel um ein lentivirales Partikel handelt.

## Revendications

1. Composition sèche comprenant :
un agent de transfection polymérique de type poly(β-amino ester) ;
des acides nucléiques codant pour toutes les protéines nécessaires pour la production d'une particule rétrovirale, les acides nucléiques étant présents sur des vecteurs différents ; et
un tampon choisi dans le groupe constitué par un tampon de phosphate de potassium, un tampon de citrate, un tampon d'acide maléique, un tampon d'acétate, un tampon de tris-(hydroxyméthyl)-aminométhane (tris), un tampon de tricine, un tampon HEPES et un tampon MOPS.

2. Composition sèche selon la revendication 1, la composition sèche étant une composition lyophilisée.

3. Composition sèche selon la revendication 1 ou la revendication 2, la composition étant exempte d'agent cryoprotecteur.

4. Composition selon l'une quelconque des revendications 1 à 3, les acides nucléiques comprenant chacun une cassette d'expression codant pour une ou plusieurs protéines rétrovirales, et les protéines pour la production d'une particule rétrovirale comprenant GAG-POL ou VSG-G.

5. Composition selon l'une quelconque des revendications 1 à 4, les acides nucléiques codant pour toutes les protéines nécessaires pour la production d'une particule rétrovirale étant distribués parmi deux vecteurs ou plus, trois vecteurs ou plus ou quatre vecteurs ou plus.

6. Composition selon l'une quelconque des revendications 1 à 5, la particule rétrovirale étant une particule lentivirale.

7. Procédé d'introduction d'un acide nucléique d'intérêt dans une cellule *in vitro,* le procédé comprenant :
a) la combinaison d'une composition selon l'une quelconque des revendications 1 à 6 avec un solvant approprié pour l'introduction d'un acide nucléique dans une cellule et comprenant l'acide nucléique d'intérêt pour produire une solution de transfection liquide ; et
b) la mise en contact de la solution de transfection liquide avec la cellule *in vitro* pour introduire l'acide nucléique dans la cellule.

8. Procédé selon la revendication 7, la concentration du tampon dans la solution de transfection liquide étant dans une plage de 50 mM à 500 mM.

9. Procédé selon la revendication 7 ou la revendication 8, l'acide nucléique d'intérêt codant pour un ARNm, une protéine ou une molécule d'ARN active.

10. Kit comprenant :
un récipient comprenant une pluralité de compartiments de réaction individuels, où chacun des compartiments de réaction comprend une composition sèche comprenant :
un agent de transfection polymérique de type poly(β-amino ester) ;
des acides nucléiques codant pour toutes les protéines nécessaires pour la production d'une particule rétrovirale, les acides nucléiques étant présents sur des vecteurs différents ; et
un tampon choisi dans le groupe constitué par un tampon de phosphate de potassium, un tampon de citrate, un tampon d'acide maléique, un tampon d'acétate, un tampon de tris-(hydroxyméthyl)-aminométhane (tris), un tampon de tricine, un tampon HEPES et un tampon MOPS.

11. Kit selon la revendication 10, comprenant en outre un vecteur comprenant une cassette de pro-expression qui comprend un site de clonage lié de manière fonctionnelle à un élément régulateur.

12. Procédé selon la revendication 7, le solvant comprenant un liquide aqueux.

13. Kit selon les revendications 11 ou 12, la particule rétrovirale étant une particule lentivirale.
